# FASCICULE DE BREVET EUROPEEN

(11) **EP 0 580 459 B1**
(45) Date de publication et mention de la délivrance du brevet: **28.03.2001**
(21) Numéro de dépôt: 93401361.6
(22) Date de dépôt: 28.05.1993
(51) Int. Cl.: C07D 233/86, C07D 235/02, C07D 233/88, C07D 233/84, C07D 233/74

(54) **Phénylimidazolidines substituées, leur application comme médicaments et les compositions pharmaceutiques les renfermant**
Substituierte Phenylimidazolidine, ihre Anwendung als Medikamente und die sie enthaltende pharmazeutische Präparate
Substituted Phenylimidazolidins, their use as medicaments and their pharmaceutical compositions containing them

(30) Priorité: 08.07.1992 FR 9208431
(43) Date de publication de la demande: 26.01.1994
(73) Titulaire: HOECHST MARION ROUSSEL, 92800 Puteaux (FR)
(72) Inventeur: Gaillard-Kelly, Martine, F-75009 Paris (FR); Goubet, François, F-75015 Paris (FR); Philibert, Daniel, F-94210 La Varenne Saint Hilaire (FR); Teutsch, Jean-Georges, F-93500 Pantin (FR)
(74) Mandataire: Vieillefosse, Jean-Claude

(56) Documents cités:
- EP-A- 0 001 813
- EP-A- 0 091 596
- EP-A- 0 436 426
- EP-A- 0 494 819
- FR-A- 2 075 751
- FR-A- 2 329 276
- US-A- 4 473 393
- US-A- 4 753 957
- US-I5- B 379 038
- CHEMICAL ABSTRACTS, vol. 81, no. 7, 19 Aoüt 1974, Columbus, Ohio, US; abstract no. 34559b, 'sumitomo' page 149 ;colonne L ; & JP-A-7 387 030 (SUMITOMO)

## Description

La présente invention concerne des phénylimidazolidines, leur application comme médicaments et les compositions pharmaceutiques les renfermant.

Dans la demande japonaise J 48087030 sont décrites des 3-phényl 2-thiohydantoïnes qui sont présentées comme inhibant la germination de certaines plantes.

Dans le brevet français 2.329.276 sont décrites des imidazolidines qui sont présentées comme possédant une activité antiandrogène. Les produits de ce brevet sont cependant différents des produits de la présente demande de brevet.

La demande de brevet européenne EP 0091596 décrit des dérivés de l'hydantoïne utiles en botanique.
La demande de brevet européenne EP 0001813 décrit des dérivés de l'imidazolidine utiles pour des préparations pharmaceutiques ayant une activité anti-androgène ou schistosomicide. Le brevet EP 0436426 décrit un procédé de préparation de dérivés de la 1-phénylimidazoline-2,5-dione.
Le brevet français 2075751 décrit des dérivés de la thiohydantoïne ayant une activité herbicide.
Le brevet US 4,473,393 décrit des dérivés de l'hydantoïne ayant une activité pesticide.
Le brevet US 4,753,957 décrit des dérivés 2,4-imidazolidine dione ayant une activité fongicide.
Le brevet US B 379038 décrit des compositions pharmaceutiques renfermant un composé 3-aryl-2-hydantoïne ayant une activité anti-arthritique.

La présente invention a donc pour objet les produits suivants :
- le 4-(4,4-diméthyl 2,5-dioxo 3-(4-hydroxy butyl) 1imidazolidinyl) 2-(trifluorométhyl) benzonitrile
- le 4-(4,4-diméthyl 3-(2-hydroxyéthyl) 5-oxo 2-thioxo 1-imidazolidinyl) 2-(trifluorométhyl) benzonitrile
- le 4-(4,4-diméthyl 3-(3-hydroxypropyl) 5-oxo 2-thioxo 1-imidazolidinyl) 2-(trifluorométhyl) benzonitrile
- le 4-(4,4-diméthyl 3-(4-hydroxybutyl) 5-oxo 2-thioxo 1-imidazolidinyl) 2-(trifluorométhyl) benzonitrile
- le 4-(4,4-diméthyl 3-(2-méthoxyéthyl) 5-oxo 2-thioxo 1-imidazolidinyl) 2-(trifluorométhyl) benzonitrile
- le 4-(4,4-diméthyl 3-(1-méthyléthyl) 5-oxo 2-thioxo 1-imidazolidinyl) 2-(trifluorométhyl) benzonitrile

La présente invention a ainsi pour objet les produits définis ci-dessus dont les noms suivent :
- le 4-[4,4-diméthyl 3-(2-hydroxyéthyl) 5-oxo 2-thioxo 1imidazolidinyl] 2-(trifluorométhyl) benzonitrile,
- le 4-(4,4-diméthyl 3-(4-hydroxybutyl) 5-oxo 2-thioxo 1-imidazolidinyl) 2-(trifluorométhyl) benzonitrile.

Les produits de la présente invention peuvent être préparés selon un procédé de préparation de produits de formule générale (I) : 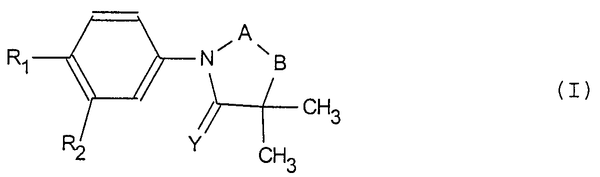 dans laquelle :
les substituants R₁, R₂, X, Y et R₃ ont les significations correspondantes aux produits de la présente invention caractérisé en ce que :
soit l'on fait agir en présence d'une base tertiaire un produit de formule (II) : 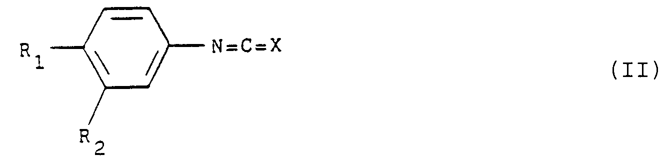 dans laquelle R₁, R₂ et X ont la signification indiquée ci-dessus, avec un produit de formule (III) : 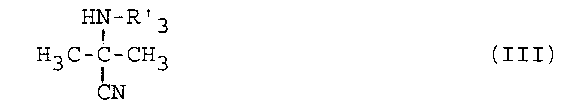 dans laquelle R'₃ a les valeurs indiquées ci-dessus pour R₃ dans lequel les éventuelles fonctions réactives sont éventuellement protégées pour obtenir un produit de formule (IV) : 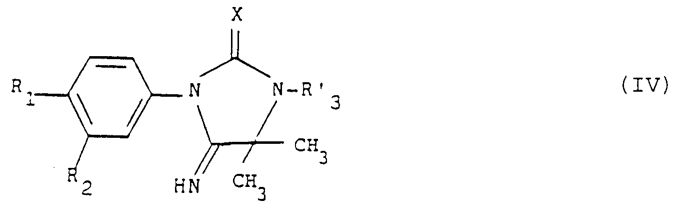 dans laquelle R₁, R₂, X et R'₃ ont la signification précédente, produits de formule (IV) que, si nécessaire ou si désiré l'on soumet à l'une quelconque ou plusieurs des réactions suivantes, dans un ordre quelconque :
a) réaction d'élimination des éventuels groupements protecteurs que peut porter R'₃;
b) réaction d'hydrolyse du groupement >C=NH en fonction cétone et le cas échéant transformation du groupement >C=S en groupement >C=O ;
c) réaction de transformation du ou des groupements >C=O en groupement >C=S ;
d) action sur les produits de formule (IV) dans laquelle R'₃ représente un atome d'hydrogène, et après hydrolyse au groupement >C=NH en fonction cétone d'un réactif de formule Hal-R"₃ dans laquelle R"₃ a les valeurs de R'₃ à l'exception de la valeur hydrogène et Hal représente un atome d'halogène pour obtenir des produits de formule (I) dans laquelle le groupement -A-B- représente le groupement 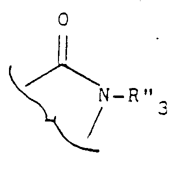 dans lesquels R"₃ a la signification indiquée précédemment puis, si désiré, action sur ces produits, d'un agent d'élimination des éventuels groupements protecteurs que peut porter R"₃ ou le cas échéant, action d'un agent d'estérification, d'amidification ou de salification,
soit l'on fait agir en présence d'une base tertiaire un produit de formule (II) : 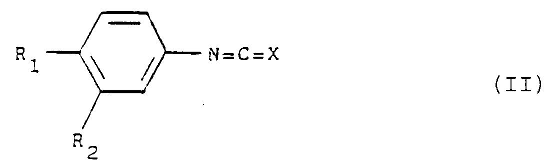 dans laquelle R₁, R₂ et X ont la signification indiquée ci-dessus, avec un produit de formule (III') : 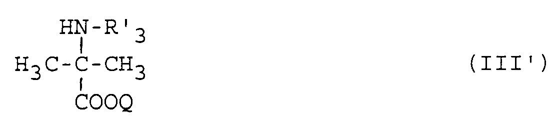 dans laquelle R'₃ a la signification indiquée ci-dessus et Q représente soit un atome de métal alcalin par exemple le sodium ou un radical alkyle renfermant de 1 à 6 atomes de carbone, pour obtenir un produit de formule (IVa) : 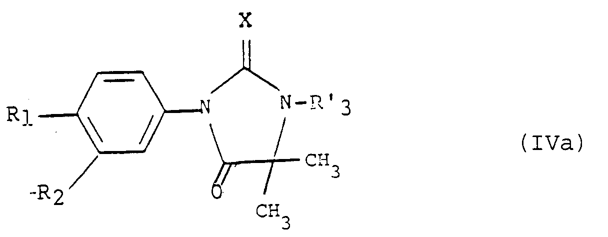 dans laquelle X, R₁, R₂ et R'₃ ont la signification indiquée ci-dessus que si désiré l'on soumet à l'une quelconque ou plusieurs des réactions suivantes, dans un ordre quelconque :
a) réaction d'élimination des éventuels groupements protecteurs que peut porter R'₃ ;
b) réaction de transformation du ou des groupements >C=O en groupement >C=S ou le cas échéant du groupement >C=S en groupement >C=O ;
c) action sur les produits de formule (IVa) dans laquelle R'₃ représente un atome d'hydrogène, d'un réactif de formule Hal-R"₃ dans laquelle R"₃ a les valeurs de R'₃ à l'exception de la valeur hydrogène et Hal représente un atome d'halogène pour obtenir des produits de formule (I) dans laquelle le groupement -A-B- représente le groupement : 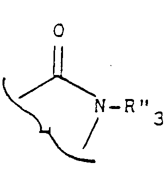 dans lesquels R"₃ a la signification indiquée précédemment puis, si désiré, action sur ces produits, d'un agent d'élimination des éventuels groupements protecteurs que peut porter R"₃ ou le cas échéant, action d'un agent d'estérification, d'amidification ou de salification,
soit l'on fait agir un réactif de formule Hal-R"₃ dans laquelle Hal et R"₃ ont les valeurs indiquées précédemment sur un produit de formule (IV') : 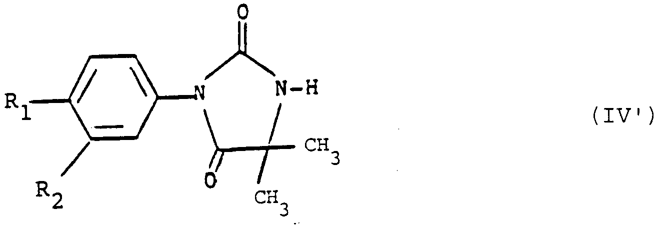 pour obtenir un produit de formule (IV") : 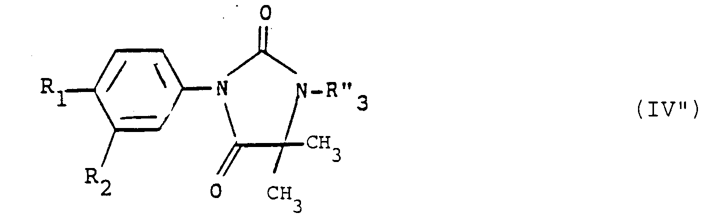 produit de formule (IV") que, si nécessaire ou si désiré l'on soumet à l'une quelconque ou plusieurs des réactions suivantes dans un ordre quelconque :
a) réaction d'élimination des éventuels groupements protecteurs que peut porter R"₃ puis le cas échéant action d'un agent d'estérification, d'amidification ou de salification ;
b) réaction de transformation du ou des groupements >C=O en groupements >C=S.

L'action des produits de formule (II) avec les produits de formule (III) est effectuée de préférence dans un solvant organique tel que le tétrahydrofuranne ou le dichloroéthane mais on peut également utiliser l'éther éthylique ou l'éther isopropylique.

On opère en présence d'une base tertiaire telle que la triéthylamine ou encore la pyridine ou la méthyléthylpyridine.

Les éventuelles fonctions réactives que peut comporter R₃ et qui sont éventuellement protégées dans le produit de formule (III), (IVa) ou (IV") sont les fonctions hydroxy ou amino. On utilise pour protéger ces fonctions des groupements protecteurs usuels. On peut citer par exemples les groupements protecteurs suivants du radical amino : tert-butyle, tert-amyle, trichloroacétyle, chloroacétyle, benzhydryle, trityle, formyle, benzyloxycarbonyle.

Comme groupement protecteur du radical hydroxy on peut citer les radicaux tels que formyle, chloroacétyle, tétrahydropyrannyle, triméthylsilyle, tert-butyl diméthylsilyle.

Il est bien entendu que la liste ci-dessus n'est pas limitative et que d'autres groupements protecteurs, par exemple connus dans la chimie des peptides peuvent être utilisés. Une liste de tels groupements protecteurs se trouve par exemple dans le brevet français BF 2.499.995 dont le contenu est incorporé ici par référence.

Les réactions éventuelles d'élimination des groupements protecteurs sont effectuées comme indiqué dans ledit brevet BF 2.499.995. Le mode préféré d'élimination est l'hydrolyse acide à l'aide des acides choisis parmi les acides chlorhydrique, benzène sulfonique ou para toluène sulfonique, formique ou trifluoroacétique. On préfère l'acide chlorhydrique.

La réaction éventuelle d'hydrolyse du groupement >C=NH en groupement cétone est également effectuée de préférence à l'aide d'un acide tel que l'acide chlorhydrique aqueux par exemple au reflux.

Lorsque l'hydrolyse du groupement >C=NH en groupement cétone est effectuée sur une molécule comportant également un groupement >C=S, celui-ci peut être transformé en groupement >C=O. Le radical OH libre que peut comporter éventuellement R₃ peut être alors transformé en radical SH.

La réaction de transformation du ou des groupements >C=O en groupement >C=S est effectuée à l'aide du réactif dit de Lawesson de formule : 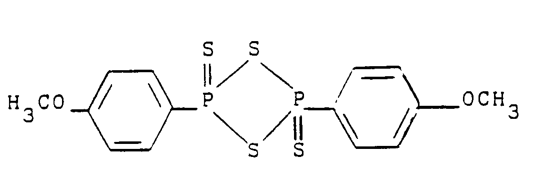 qui est un produit commercialisé par exemple par la firme FLUKA et dont l'utilisation est décrite par exemple dans la publication : Bull. Soc. Chim. Belg. vol 87, N° 3, (1987) p. 229.

Lorsque l'on veut transformer deux fonctions >C=O en deux fonctions >C=S on opère en présence d'un excès de réactif de Lawesson. Il en est de même lorsque l'on part d'une molécule comportant une fonction >C=S et une fonction >C=O et que l'on veut transformer ladite fonction >C=O en fonction >C=S.

Par contre lorsque l'on part d'une molécule comportant deux fonctions >C=O et que l'on veut obtenir un produit ne comportant qu'une seule fonction >C=S. On opère en présence d'un déficit de réactif de Lawesson. On obtient alors en général un mélange de trois produits : chacun des deux produits comportant une fonction >C=O et une fonction >C=S et le produit comportant deux fonctions >C=S. Ces produits peuvent être ensuite séparés par les méthodes usuelles telles que la chromatographie.

L'action sur les produits de formules (IV), (IVa) ou (IV') du réactif de formule Hal-R"₃ est effectuée en présence d'une base forte telle que l'hydrure de sodium ou de potassium. On peut opérer par réaction de transfert de phase en présence de sels d'ammonium quaternaires tels que le tert-butyl ammonium.
- Les groupements protecteurs que peut porter le substituant R"₃ pouvant être par exemple un de ceux précédemment cités pour R₃. Les réactions d'élimination des groupements protecteurs s'effectuent dans les conditions indiquées ci-dessus.
   Un exemple d'élimination du groupement terbutyldiméthylsilyle au moyen de l'acide chlorhydrique est donné ci-après dans les exemples.
- L'estérification éventuelle des produits de formule (I) dans laquelle R"₃ comporte un radical OH libre est effectuée dans des conditions classiques. On peut utiliser par exemple un acide ou un dérivé fonctionnel, par exemple un anhydride tel que l'anhydride acétique en présence d'une base telle que la pyridine.
   L'estérification ou la salification éventuelle des produits de formule (I) dans laquelle R"₃ représente un groupement COOH est effectuée dans les conditions classiques connues de l'homme du métier.
- L'amidification éventuelle des produits de formule (I) dans laquelle R"₃ comporte un radical COOH est effectuée dans des conditions classiques. On peut utiliser une amine primaire ou secondaire sur un dérivé fonctionnel de l'acide par exemple un anhydride symétrique ou mixte.

Les produits de la présente invention peuvent également être préparés selon un procédé de préparation des produits de formule (I") : 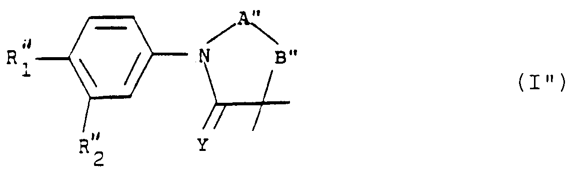 dans laquelle R"₁, R"₂, -A"-B"- ont les significations indiquées ci-dessus pour R₁, R₂ et -A-B- ce procédé étant caractérisé en ce que l'on fait réagir un produit de formule (V) : 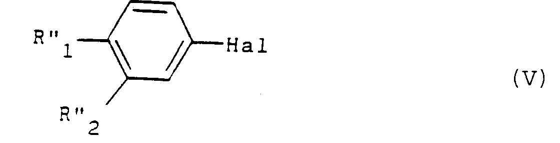 dans laquelle R"₁ et R"₂ ont les significations précédentes et Hal représente un atome d'halogène avec un produit de formule (VI) : 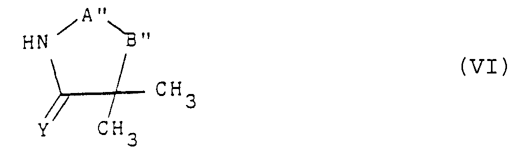 dans laquelle -A"-B"- et Y ont la signification indiquée ci-dessus, la réaction s'effectuant en présence d'un catalyseur et éventuellement d'un solvant.

En ce qui concerne les produits de formule (V), le terme Hal désigne de préférence l'atome de chlore, mais peut aussi représenter un atome de brome ou d'iode.

Le rôle du catalyseur est vraisemblablement de piéger l'halogénure d'hydrogène qui se dégage et ainsi de faciliter la réaction de condensation du produit de formule (V) avec le produit de formule (VI) pour donner le produit recherché.

Plus précisément dans le procédé tel que défini ci-dessus le catalyseur est un métal sous forme native ou oxydée ou une base.

Le catalyseur utilisé peut être un métal sous forme native, sous forme d'oxyde métallique ou encore sous forme de sels métalliques. Le catalyseur peut également être une base. Quand le catalyseur utilisé est un métal, ce métal peut être du cuivre ou du nickel.

Les sels métalliques peuvent être un chlorure ou un acétate.

Quand le catalyseur est une base, cette base peut être par exemple la soude ou la potasse et on peut, si désiré, ajouter au milieu réactionnel du diméthylsulfoxyde.

Plus précisément dans le procédé tel que défini ci-dessus le catalyseur est choisi parmi l'oxyde cuivreux, l'oxyde cuivrique, le cuivre sous forme native et une base telle que la soude ou la potasse.

Le cuivre sous forme native utilisé comme catalyseur est préférentiellement sous forme de poudre.

Dans un tel procédé tel que défini ci-dessus le catalyseur est particulièrement l'oxyde cuivreux.

Le solvant utilisé est préférentiellement choisi parmi des éthers à haut point d'ébullition tels que, par exemple, l'oxyde de phényle, le diglyme, le triglyme et le diméthylsulfoxyde mais peut être également, par exemple, une huile à haut point d'ébullition telle que la paraffine ou la vaseline.

Plus particulièrement le procédé tel que défini ci-dessus est caractérisé en ce que l'on opère en présence d'un solvant de type éther tel que l'oxyde de phényle, le diglyme, le triglyme ou le diméthylsulfoxyde.

Tout particulièrement dans le procédé tel que défini ci-dessus, le solvant utilisé est l'oxyde de phényle ou le triglyme.

Le procédé de préparation du produit recherché, défini ci-dessus peut être réalisé sous pression ou à la pression atmosphérique, à une température préférentiellement élevée.

Le procédé tel que défini ci-dessus peut être caractérisé en ce que la réaction est réalisée à une température supérieure à 100°C et de préférence supérieure à 150°C.

Le procédé tel que défini ci-dessus est plus précisément caractérisé en ce que la réaction est réalisée pendant plus de 2 heures.

Le procédé tel que défini ci-dessus est très précisément caractérisé en ce que la réaction est réalisée en présence d'oxyde cuivreux, dans le triglyme, à une température supérieure ou égale à 200°C et pendant plus de 3 heures.

Les produits objets de la présente invention sont doués de propriétés pharmacologiques intéressantes ; on a constaté notamment qu'ils inhibaient les effets des androgènes sur les récepteurs périphériques.

Des tests donnés dans la partie expérimentale illustrent cette activité anti-androgène.

Du fait de cette activité anti-androgène, les produits de l'invention peuvent être utilisés en thérapeutique chez les adultes sans avoir à redouter certains effets d'une castration chimique.

Ces propriétés rendent les produits de la présente invention utilisables comme médicaments pour le traitement des adénomes et des néoplasies de la prostate ainsi que pour lutter contre l'hypertrophie bénigne de la prostate.

Ces propriétés rendent les produits de la présente invention également utilisables dans le traitement des tumeurs bénignes ou malignes dont les cellules contiennent notamment des récepteurs androgènes. On peut en particulier citer principalement les cancers du sein, du cerveau, de la peau et des ovaires mais également les cancers de la vessie, du système lymphatique, du rein, du foie.

Les produits de l'invention trouvent également leur utilisation dans le traitement de l'hirsutisme, de l'acné, de la seborrhée, de l'alopécie androgénique, de l'hyperpilosité ou hirsutisme.

Les produits de la présente invention peuvent donc être utilisés en dermatologie : ils peuvent être utilisés seuls ou en association. Ils peuvent être associés notamment avec un produit antibiotique tels que les dérivés de l'acide azélaique, fusidique, l'érythromycine ou avec un dérivé des rétinoïdes pour le traitement de l'acné, ou avec un inhibiteur de la 5α-réductase tel que le (5α,17β))-1,1-diméthyléthyl 3-oxo 4-aza-androst-1-ène 17-carboxamide (ou Finastéride Merck, 11ème ed.) ou l'acide azélaïque ou un agent bloquant des récepteurs androgènes pour le traitement de l'acné, de l'alopécie ou de l'hirsutisme, ou avec un produit stimulant la croissance des cheveux tel que le Minoxidil pour le traitement de l'alopécie.

Les produits de la présente invention peuvent également être utilisés dans le domaine vétérinaire.

Les produits de la présente invention, sous forme de produits radioactifs peuvent encore être utilisés en diagnostic comme marqueurs spécifiques des récepteurs androgènes. Comme produits radioactifs, on peut utiliser par exemple, des produits marqués au tritium, au carbone 14 ou encore à l'iode 125.

L'invention a donc pour objet l'application, à titre de médicaments, des produits de la présente invention pharmaceutiquement acceptables.

L'invention a particulièrement pour objet l'application à titre de médicaments, des produits dont les noms suivent :
- le 4-[4,4-diméthyl 3-(2-hydroxyéthyl) 5-oxo 2-thioxo 1-imidazolidinyl] 2-(trifluorométhyl) benzonitrile,
- le 4-(4,4-diméthyl 2,5-dioxo 3-(4-hydroxybutyl) 1-imidazolidinyl) 2-(trifluorométhyl) benzonitrile,
- le 4-(4,4-diméthyl 3-(4-hydroxybutyl) 5-oxo 2-thioxo 1-imidazolidinyl) 2-(trifluorométhyl) benzonitrile.

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments ci-dessus.

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffini-ques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

Les produits de formule (II) utilisés au départ de l'invention peuvent être obtenus par action du phosgène lorsque X représente un atome d'oxygène ou du thiophosgène lorsque X représente un atome de soufre sur l'amine correspondante de formule (A) :

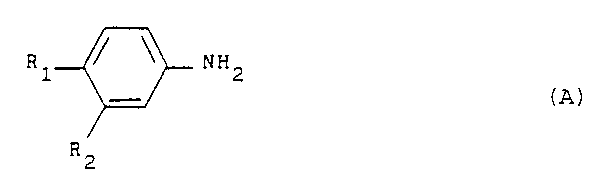

Un exemple d'une telle préparation est donné ci-après dans la partie expérimentale. Un produit de ce type est décrit également dans le brevet français BF 2.329.276.

Les amines de formule (A) sont décrites dans le brevet européen EP 0.002.892 ou le brevet français BF 2.142.804.

Les produits de formule (III) ou (III') sont connus ou peuvent être préparés à partir de la cyanhydrine correspondante selon le procédé décrit dans la publication : J. Am. Chem. Soc. (1953), 75, 4841.

Les produits de formule (III) dans lesquels R'₃ est différent d'un atome d'hydrogène peuvent être obtenus par action d'un produit de formule R"₃-Hal sur le 2-cyano 2-amino propane dans les conditions énoncées ci-dessus pour l'action de R"₃ Hal sur les produits de formule (IV). Un exemple de préparation de ce type est décrit dans la référence :
- Jilek et Coll. Collect. Czech. Chem. Comm. 54(8) 2248 (1989).

Les produits de formule (IV') sont décrits dans le brevet français BF 2.329.276.

Les produits de départ de formules (V) et (VI), sur lesquels s'exerce un procédé pour l'obtention des produits de formule (I), sont connus et disponibles dans le commerce ou peuvent être préparés selon des méthodes connues de l'homme de métier.

La préparation de produits de formule (VI) est décrite notamment dans les publications suivantes :
- Zhur. Préklad. Khim. 28, 969-75 (1955) (CA 50, 4881a, 1956)
- Tétrahédron 43, 1753 (1987)
- J. Org. Chem. 52, 2407 (1987)
- Zh. Org. Khim. 21, 2006 (1985)
- J. Fluor. Chem. 17, 345 (1981)
   ou dans les brevets :
- allemand DRP 637.318 (1935)
- européen EP 0.130.875
- japonais JP 81.121.524.

Les produits de formule (VI) qui sont des dérivés de l'hydantoïne sont largement utilisés et cités dans la littérature comme par exemple dans les articles suivants :
- J. Pharm. Pharmacol., 67, Vol. 19(4), p. 209-16 (1967)
- Khim. Farm. Zh., 67, Vol. 1 (5) p. 51-2
- Brevet allemand 2.217.914
- Brevet européen 0.091.596
- J. Chem. Soc. Perkin. Trans. 1, p. 219-21 (1974).

Parmi les fonctions réactives protégées on peut citer les fonctions hydroxyle et amino. Ces fonctions peuvent être protégées comme indiqué ci-dessus pour le substituant R₃.

### EXEMPLE 1 : 4-(4,4-diméthyl 2,5-dioxo 3-(4-hydroxy butyl) 1-imidazolidinyl) 2-(trifluorométhyl) benzonitrile

### a) Condensation

A une suspension de 104 mg d'hydrure de sodium dans 0,8 cm³ de diméthylformamide, on ajoute 600 mg de 4-(4,4-diméthyl-2,5-dioxo-1-imidazolidinyl) -2- (trifluorométhyl)-benzonitrile obtenu comme à l'exemple 8 de la demande de brevet européen n° 0 494 819 dans 5 cm³ de diméthylformamide en maintenant la température inférieure à 20°C. Après 10 minutes d'agitation, on ajoute 445 mg de 4-chloro t-butyl diméthylsilyléther et 300 mg d'iodure de sodium. On chauffe 16 heures à 50°C, refroidit à température ambiante, ajoute 87 mg d'hydrure de sodium puis de nouveau 400 mg de l'éther chloré et 267 mg d'hydrure de sodium, et chauffe 1 heure supplémentaire. On ramène à température ambiante, verse sur 60 cm³ d'eau contenant 600 mg de phosphate monopotassique. On extrait à l'éther, lave la phase organique à l'eau, sèche et évapore le solvant. On chromatographie le résidu sur silice (éluant chlorure de méthylène-acétone (99-1)), recueille 526 mg de produit utilisé tel quel pour le stade suivant le clivage.

On mélange le produit obtenu ci-dessus dans 5 cm³ de méthanol et 1,5 cm³ d'acide chlorhydrique 2N. On agite 40 minutes à température ambiante, verse sur 30 cm³ d'eau, extrait au chlorure de méthylène, lave la phase organique à l'eau, sèche et évapore le solvant. Après chromatographie du résidu sur silice (éluant chlorure de méthylène-acétone (9-1) on récupère les fractions rf = 0,15 et après recristallisation dans l'éther isopropylique, on obtient 307 mg de produit attendu. F = 102-103°C.

| Analyse C₁₇H₁₈F₃N₃O₃ = 369,35 | | | | |
|---|---|---|---|---|
| | C % | H % | F % | N % |
| Calculé | 55,28 | 4,91 | 15,43 | 11,38 |
| Trouvé | 55,2 | 4,9 | 15,3 | 11,1 |

| Spectre IR (CHCl₃) | |
|---|---|
| OH | 3628 cm⁻¹ |
| C≡N | 2236 cm⁻¹ |
| C=O | 1778-1724 cm⁻¹ |
| Aromatiques | 1615-1575-1505 cm⁻¹ |

### Préparation du 4-chloro t-butyl diméthylsilyléther utilisé au départ de l'exemple 58.

On mélange sous agitation 9,9 cm³ de 4-chloro 1-butanol et 24,3 g d'imidazole dans 50 cm³ de tétrahydrofuranne. On ajoute goutte à goutte à une température inférieure à 20°C, 2,82 g de chlorure de terbutyldiméthylsilyle, dans 20 cm³ de tétrahydrofuranne, agite 18 heures à température ambiante, essore, rince au tétrahydrofuranne et élimine le solvant sous pression réduite. On purifie le résidu par chromatographie sur silice (éluant cyclohexane-acétate d'éthyle (95-5)) et recueille 17,5 g de produit attendu.

### EXEMPLE 2 : 4-(4,4-diméthyl 3-(2-hydroxyéthyl) 5-oxo 2-thioxo 1-imidazolidinyl) 2-(trifluorométhyl) benzonitrile

### A/ 4-(4,4-diméthyl 3-(2-hydroxyéthyl) 5-imino 2-thioxo 1-imidazolidinyl) 2-(trifluorométhyl) benzonitrile

### a) Préparation de l'isothiocyanate

A une solution de 22 cm³ d'eau distillée et 1 cm³ de thiophosgène on ajoute lentement 2,23 g de 1-trifluorométhyl-4-amino benzonitrile (préparé selon EP 0002892) on agite pendant 1 heure, extrait avec du chloroforme, lave à l'eau salée, sèche et évapore à sec sous pression réduite, on obtient 3 g de produit utilisé tel quel pour l'obtention de l'imine.

### b) Obtention de l'imine

On mélange à température ambiante 5 g de l'isothiocyanate préparée ci-dessus dans 37 cm³ de tétrahydrofuranne en présence du 1,5 cm³ de triéthylamine et ajoute en une fois 2,8 g de 2-[(2-hydroxy éthyl) amino] 2-méthyl propane nitrile, préparé comme indiqué dans la préparation donnée à l'exemple 22 de la demande de brevet européen n° 0 494 819, en solution dans 10 cm³ de tétrahydrofuranne. La température s'élève spontanément à 34°C. On laisse revenir à température ambiante en maintenant 1 heure sous agitation, évapore le solvant et chromatographie le résidu sur silice (éluant chlorure de méthylène-méthanol (7-3)).

On obtient 5,87 g de produit attendu (F = 181°C, après cristallisation dans l'isopropanol).

### B/ 4-(4,4-diméthyl 3-(2-hydroxyéthyl) 5-oxo 2-thioxo 1-imidazolidinyl) 2-(trifluorométhyl) benzonitrile

On chauffe 1 heure au reflux 4,6 g de produit préparé ci-dessous en A dans 65 cm³ de méthanol en présence de 10 cm³ d'acide chlorhydrique 2N. On refroidit à température ambiante et verse sur 300 cm³ d'eau glacée. On extrait à l'acétate d'éthyle, lave la phase organique à l'eau salée, sèche et évapore le solvant. On chromatographie le résidu sur silice (acétate d'éthyle-cyclohexane (1-1)), recueille les fractions
rf = 0,14 et obtient après cristallisation dans le chlorure de méthylène et cyclohexane 4,37 g de produit attendu.
F = 130°C.

| Analyse C₁₅H₁₄F₃N₃O₂S=357,36 | | | | | |
|---|---|---|---|---|---|
| | C % | H % | F % | N % | S % |
| Calculé | 50,42 | 3,95 | 15,95 | 11,76 | 8,97 |
| Trouvé | 50,3 | 3,9 | 15,9 | 11,6 | 8,9 |

| Spectre IR (CHCl₃) | |
|---|---|
| OH | 3626 cm⁻¹ |
| C≡N | 2236 cm⁻¹ |
| C=O | 1763 cm⁻¹ |
| Aromatiques | 1615-1578-1504 cm⁻¹ |

### EXEMPLE 3: 4-(4,4-diméthyl 3-(3-hydroxypropyl) 5-oxo 2-thioxo 1-imidazolidinyl) 2-(trifluorométhyl) benzonitrile

### A/ 4-(4,4-diméthyl 3-(3-hydroxypropyl) 5-imino 2-thioxo 1-imidazolidinyl) 2-(trifluorométhyl) benzonitrile

En opérant comme à l'exemple 22 de la demande de brevet européen n° 0 494 819 en utilisant au départ 2 g d'isothiocyanate préparé comme indiqué à l'exemple 13a) et 1,2 g de l'aminonitrile approprié, on obtient 1,70 g de produit attendu.
rf = 0,25 (chlorure de méthylène-acétone (65-35)).

| Spectre IR (CHCl₃) | |
|---|---|
| OH | 3630 cm⁻¹ |
| =NH | 3314-1676 cm⁻¹ |
| C≡N | 2235 cm⁻¹ |
| Aromatiques | 1614-1578-1481 cm⁻¹ |

### B/ 4-(4,4-diméthyl 3-(3-hydroxypropyl) 5-oxo 2-thioxo 1-imidazolidinyl) 2-(trifluorométhyl) benzonitrile

En opérant comme à l'exemple 2 à partir de 240 mg de produit obtenu ci-dessus en A /, on obtient 226 mg de produit attendu. F = 149-150°C.
Rf = 0,32 (éluant chlorure de méthylène-acétone (75-25)).

| Spectre IR (CHCl₃) | |
|---|---|
| OH | 3626 cm⁻¹ |
| C=O | 1763 cm⁻¹ |
| C≡N | 2236 cm⁻¹ |
| Aromatiques | 1615-1580-1504-1483 cm⁻¹ |

### EXEMPLE 4 : 4-(4,4-diméthyl 3-(4-hydroxybutyl) 5-oxo 2-thioxo 1-imidazolidinyl) 2-(trifluorométhyl) benzonitrile

### A/ 4-(4,4-diméthyl 3-(4-hydroxybutyl) 5-imino 2-thioxo 1-imidazolidinyl) 2-(trifluorométhyl) benzonitrile

En opérant comme à l'exemple 22 de la demande de brevet européen n° 0 494 819, en utilisant au départ 2 g d'isothiocyanate et 1,38 g de l'aminonitrile approprié, on obtient 2,08 g de produit attendu.
rf = 0,25 (chlorure de méthylène-acétone (65-35)).

| Spectre IR (CHCl₃) | |
|---|---|
| OH | 3630 cm⁻¹ |
| =NH | 3314-1675 cm⁻¹ |
| C≡N | 2235 cm⁻¹ |
| Aromatiques | 1614-1577-1504 cm⁻¹ |

### B/ 4-(4,4-diméthyl 3-(4-hydroxybutyl) 5-oxo 2-thioxo 1-imidazolidinyl) 2-(trifluorométhyl) benzonitrile

En opérant comme au B/ de l'exemple 2, à partir de 300 mg de produit obtenu ci-dessus en A/, on obtient 236 mg de produit attendu. F = 78-79°C.
Rf = 0,31 (éluant chlorure de méthylène-acétone (75-25)).

| Spectre IR (CHCl₃) | |
|---|---|
| OH | 3624 cm⁻¹ |
| C=O | 1762 cm⁻¹ |
| C≡N | 2237 cm⁻¹ |
| Aromatiques | 1615-1580-1504 cm⁻¹ |

| Spectre UV (EtOH) | |
|---|---|
| Max. 232 nm | ε= 19500 |
| Max. 254 nm | ε= 24000 |
| Infl. 266 nm | |

### EXEMPLE 5 : 4-(4,4-diméthyl 3-(2-méthoxyéthyl) 5-oxo 2-thioxo 1-imidazolidinyl) 2-(trifluorométhyl) benzonitrile

### A/ 4-(4,4-diméthyl 3-(2-méthoxyéthyl) 5-imino 2-thioxo 1-imidazolidinyl) 2-(trifluorométhyl) benzonitrile

En opérant comme à l'exemple 22 de la demande de brevet européen n° 0 494 819, en utilisant au départ 2,5 g d'isothiocyanate et 1,56 g d'aminonitrile appropriés, on obtient 2,36 g de produit attendu.
Rf = 0,23 (chlorure de méthylène-acétone (92,5-7,5)).

| Spectre IR (CHCl₃) | |
|---|---|
| =NH | 3314 cm⁻¹ |
| C≡N | 2236 cm⁻¹ |
| Aromatiques | 1614-1578-1504 cm⁻¹ |
| C=N | 1675 cm⁻¹ |

### B/ 4-(4,4-diméthyl 3-(2-méthoxyéthyl) 5-oxo 2-thioxo 1-imidazolidinyl) 2-(trifluorométhyl) benzonitrile

En opérant comme au B/ de l'exemple 2, à partir du produit obtenu ci-dessus en A/, on obtient le produit attendu.
F = 98-99°C.
Rf = 0,32 (éluant chlorure de méthylène-acétone (99-1))

| Spectre IR (CHCl₃) | |
|---|---|
| C=O | 1757 cm⁻¹ |
| C≡N | 2236 cm⁻¹ |
| Aromatiques | 1615-1580-1504 cm⁻¹ |

| Spectre UV (EtOH) | |
|---|---|
| Max. 232 nm | ε= 18200 |
| Max. 254 nm | ε= 22400 |
| Inf. 265 nm | |

### EXEMPLE 6 : 4-(4,4-diméthyl 3-(1-méthyléthyl) 5-oxo 2-thioxo 1-imidazolidinyl) 2-(trifluorométhyl) benzonitrile

### A/ 4-(4,4-diméthyl 3-(1-méthyléthyl) 5-imino 2-thioxo 1-imidazolidinyl) 2-(trifluorométhyl) benzonitrile

En opérant comme à l'exemple 22 de la demande de brevet européen n° 0 494 819, en utilisant au départ 2,5 g d'isothiocyanate et 1,32 g de l'aminonitrile approprié, on obtient 880 mg de produit attendu.
rf = 0,20 (chlorure de méthylène-acétone (96-4)).

| Spectre IR (CHCl₃) | |
|---|---|
| =NH | 3310-1675 cm⁻¹ |
| C≡N | 2236 cm⁻¹ |
| Aromatiques | 1614-1580-1504 cm⁻¹ |

### B/ 4-(4,4-diméthyl 3-(1-méthyléthyl) 5-oxo 2-thioxo 1-imidazolidinyl) 2-(trifluorométhyl) benzonitrile

En opérant comme au B/de l'exemple 2, à partir de 880 mg de produit obtenu ci-dessus en A/ et 35 cm³ d'acide chlorhydrique 6N et après extraction au chloroforme, on obtient 744 mg de produit attendu. F = 203-204°C.
Rf = 0,45 (éluant cyclohexane-acétate d'éthyle (1-1)).

| Spectre IR (CHCl₃) | |
|---|---|
| OH | 3626 cm⁻¹ |
| C=O | 1753 cm⁻¹ |
| C=N | 2232 cm⁻¹ |
| Aromatiques | 1615-1580-1504 cm⁻¹ |

| Spectre UV (EtOH) | |
|---|---|
| Max. 232 nm | ε = 18900 |
| Max. 235 nm | ε = 22500 |
| Inf. 273 nm | |

### EXEMPLE 7:

On a préparé des comprimés ayant la composition suivante:
- 4-(4,4-diméthyl 2,5-dioxo 3-(4-hydroxy butyl) 1-imidazolidinyl) 2-(trifluorométhyl) benzonitrile 100 mg
- Excipient q.s. pour un comprimé terminé à 300 mg
(Détail de l'excipient : lactose, amidon, talc, stéarate de magnésium) .

### ETUDE PHARMACOLOGIQUE DES PRODUITS DE L'INVENTION

### 1) Etude de l'affinité des produits de l'invention pour le récepteur androgènₑ

### Récepteur androgène._{T}

Des rats mâles Sprague. Dawley EOPS de 180-200 g, castrés de 24 heures, sont sacrifiés, les prostates prélevées, pesées et homogénéisées à 0°C à l'aide d'un potter verre-verre, dans une solution tamponnée (Tris lOmM, saccharose 0,25M, PMSF (phénylméthanesulfonylfluoride) 0,1mM, Molybdate de sodium 20mM, HCl pH 7,4 ; auxquels on ajoute extemporanément 2mM de DTT (DL dithiothreitol), à raison de 1 g de tissu pour 8 ml de tampon.

L'homogénat est ensuite ultracentrifugé à 0°C, 30 minutes à 209 000 g. Des aliquotes du surnageant obtenu (=cytosol), sont incubées 30 minutes et 24 heures à 0°C, avec une concentration constante (T) de Testostérone tritiée et en présence de concentrations croissantes (0 à 2500.10⁻⁹M), soit de testostérone froide, soit des produits à tester. La concentration de Testostérone tritiée liée (B) est ensuite mesurée dans chaque incubat par la méthode d'adsorption au charbon-dextran.

### Calcul de l'affinité relative de liaison (ARL).

On trace les 2 courbes suivantes : le pourcentage de l'hormone tritiée liée B/T en fonction du logarithme de la concentration de l'hormone de référence froide et B/T en fonction du logarithme de la concentration du produit froid testé. On détermine la droite d'équation I₅₀=(B/Tmax + B/Tmin)/2.
B/T max= % de l'hormone tritiée liée pour une incubation de cette hormone tritiée à la concentration (T).
B/T min= % de l'hormone tritiée liée pour une incubation de cette hormone tritiée à la concentration (T) en présence d'un grand excès d'hormone froide (2500.10⁻⁹M) .

Les intersections de la droite I₅₀ et des courbes, permettent d'évaluer les concentrations de l'hormone de référence froide (CH) et du produit froid testé (CX) qui inhibent de
50 % la liaison de l'hormone tritiée sur le récepteur. L'affinité relative de liaison (ARL) du produit testé est déterminé par l'équation ARL=100 (CH)/(CX).

On obtient les résultats suivants exprimés en ARL.

Produit de référence (Testostérone) : 100

| Produit des exemples | Incubation : 24 heures |
|---|---|
| 1 | 31 |
| 2 | 163 |
| 4 | 300 |
| 5 | 81 |
| 6 | 28 |

### 2) Détermination de l'activité androgène ou anti-androgène des produits de l'invention à l'aide du dosage de l'ornithine décarboxylase.

### - Protocole de traitement

Des souris mâles SWISS âgées de 6 semaines, et castrées de 24 heures, reçoivent par voie orale ou percutanée les produits à étudier (suspension en méthyl cellulose à 0,5 % ou en solution dans l'éthanol), simultanément avec une injection sous-cutanée de Propionate de testostérone 3 mg/kg (solution dans l'huile de maïs) pour déterminer l'activité anti-androgène. L'activité agoniste est déterminée en l'absence de propionate de testostérone.

Le Propionate de testostérone est administré sous un volume de 10 ml/kg.

20 heures après les traitements, les animaux sont sacrifiés, les reins prélevés, puis homogénéisés à 0°C , à l'aide d'un broyeur téflon-verre dans 10 volumes de tampon Tris-HCl 50 mM (pH 7,4) contenant 250 uM de phosphate de pyridoxal, 0,1 mM EDTA, et 5 mM de dithiothreitol. L'homogenat est ensuite centrifugé à 209000 g pendant 30 mn.

### - Principe de dosage

A 37°C, l'ornithine décarboxylase rénale transforme un mélange isotopique d'ornithine froide et d'ornithine tritiée en putrescine froide et putrescine tritiée.

La putrescine est ensuite recueillie sur des papiers sélectifs, échangeurs d'ions. Après séchage, l'excès d'ornithine tritiée et froide non transformée est éliminé, par 3 lavages d'ammoniaque 0,1 M. Les papiers sont séchés, puis la radioactivité est comptée après addition de scintillant Aqualite.

Les résultats sont exprimés en fmoles (10⁻¹⁵ M) de putrescine tritiée formée/heure/mg de protéines.

Les résultats sont exprimés en % d'inhibition de l'ODL des témoins ne recevant que le propionate de testostérone.
**Test A** : les produits sont administrés par voie percutanée à 1,5 mg/kg sous un volume de 10 µl.
**Test B** : les produits sont administrés par voie orale à 1 mg/kg.
**Test C** : les produits sont administrés par voie orale à 3 mg/kg.

| Produits des exemples | Test A | ODL Test B | Test C |
|---|---|---|---|
| 1 | 40 | 36 | |
| 2 | 32 | | 67 |
| 3 | 41 | | |
| 4 | 78 | | |
| 5 | 62 | | |
| 6 | 35 | | |

**Conclusion** : Les tests indiqués ci-dessus montrent que les produits de l'invention testés possèdent une forte activité anti-androgène et sont dénués d'activité agoniste.

## Revendications

1. Les produits suivants :
- le 4-(4,4-diméthyl 2,5-dioxo 3-(4-hydroxy butyl) 1-imidazolidinyl) 2-(trifluorométhyl) benzonitrile
- le 4-(4,4-diméthyl 3-(2-hydroxyéthyl) 5-oxo 2-thioxo 1-imidazolidinyl) 2-(trifluorométhyl) benzonitrile
- le 4-(4,4-diméthyl 3-(3-hydroxypropyl) 5-oxo 2-thioxo 1-imidazolidinyl) 2-(trifluorométhyl) benzonitrile
- le 4-(4,4-diméthyl 3-(4-hydroxybutyl) 5-oxo 2-thioxo 1-imidazolidinyl) 2-(trifluorométhyl) benzonitrile
- le 4-(4,4-diméthyl 3-(2-méthoxyéthyl) 5-oxo 2-thioxo 1-imidazolidinyl) 2-(trifluorométhyl) benzonitrile
- le 4-(4,4-diméthyl 3-(1-méthyléthyl) 5-oxo 2-thioxo 1-imidazolidinyl) 2-(trifluorométhyl) benzonitrile.

2. Les produits définis à la revendication 1, dont les noms suivent :
- le 4-[4,4-diméthyl 3-(2-hydroxyéthyl) 5-oxo 2-thioxo 1-imidazolidinyl] 2-(trifluorométhyl) benzonitrile,
- le 4-(4,4-diméthyl 3-(4-hydroxybutyl) 5-oxo 2-thioxo 1-imidazolidinyl) 2-(trifluorométhyl) benzonitrile.

3. A titre de médicaments, les produits tels que définis à la revendication 1, pharmaceutiquement acceptables.

4. Les compositions pharmaceutiques contenant, à titre de principe actif, l'un au moins des médicaments tels que définis à la revendication 3.

## Patentansprüche

1. Die folgenden Produkte:
- 4-[4,4-Dimethyl-2,5-dioxo-3-(4-hydroxybutyl)-1-imidazolidinyl] 2-(trifluormethyl)-benzonitril,
- 4-[4,4-Dimethyl-3-(2-hydroxyethyl)-5-oxo-2-thioxo-1-imidazolidinyl]-2-(trifluormethyl)-benzonitril,
- 4-[4,4-Dimethyl-3-(3-hydroxypropyl)-5-oxo-2-thioxo-1-imidazolidinyl]-2-(trifluormethyl)-benzonitril,
- 4-[4,4-Dimethyl-3-(4-hydroxybutyl)-5-oxo-2-thioxo-l-imidazolidinyl]-2-(trifluormethyl)-benzonitril,
- 4-[4,4-Dimethyl-3-(2-methoxyethyl)-5-oxo-2-thioxo-1-imidazolidinyl]-2-(trifluormethyl)-benzonitril,
- 4-[4,4-Dimethyl-3-(1-methylethyl)-5-oxo-2-thioxo-1-imidazolidinyl]-2-(trifluormethyl)-benzonitril.

2. Die in Anspruch 1 definierten Produkte mit den folgenden Namen:
- 4-[4,4-Dimethyl-3-(2-hydroxyethyl)-5-oxo-2-thioxo-l-imidazolidinyl]-2-(trifluormethyl)-benzonitril,
- 4-[4,4-Dimethyl-3-(4-hydroxybutyl)-5-oxo-2-thioxo-l-imidazolidinyl]-2-(trifluormethyl)-benzonitril.

3. Als Arzneimittel die pharmazeutisch akzeptablen Produkte wie in Anspruch 1 definiert.

4. Pharmazeutische Zusammensetzungen, enthaltend als Wirkstoff mindestens eines der wie in Anspruch 3 definierten Arzneimittel.

## Claims

1. The following products:
- 4-(4,4-dimethyl 2,5-dioxo 3-(4-hydroxy butyl) 1-imidazolidinyl) 2-(trifluoromethyl) benzonitrile
- 4-(4,4-dimethyl 3-(2-hydroxyethyl) 5-oxo 2-thioxo 1-imidazolidinyl) 2-(trifluoromethyl) benzonitrile
- 4-(4,4-dimethyl 3-(3-hydroxypropyl) 5-oxo 2-thioxo 1-imidazolidinyl) 2-(trifluoromethyl) benzonitrile
- 4-(4,4-dimethyl 3-(4-hydroxybutyl) 5-oxo 2-thioxo 1-imidazolidinyl) 2-(trifluoromethyl) benzonitrile
- 4-(4,4-dimethyl 3-(2-methoxyethyl) 5-oxo 2-thioxo 1-imidazolidinyl) 2-(trifluoromethyl) benzonitrile
- 4-(4,4-dimethyl 3-(1-methylethyl) 5-oxo 2-thioxo 1-imidazolidinyl) 2-(trifluoromethyl) benzonitrile.

2. The products defined in claim 1, the names of which follow:
- 4-[4,4-dimethyl 3-(2-hydroxyethyl) 5-oxo 2-thioxo 1-imidazolidinyl] 2-(trifluoromethyl) benzonitrile,
- 4-(4,4-dimethyl 3-(4-hydroxybutyl) 5-oxo 2-thioxo 1-imidazolidinyl) 2-(trifluoromethyl) benzonitrile.

3. As medicaments, the pharmaceutically acceptable products as defined in claim 1,.

4. The pharmaceutical compositions containing, as active ingredient, at least one of the medicaments as defined in claim 3.
